# FASCICULE DE BREVET EUROPEEN

(11) **EP 4 133 277 B1**
(45) Date de publication et mention de la délivrance du brevet: **14.08.2024**
(21) Numéro de dépôt: 21723881.5
(22) Date de dépôt: 08.04.2021
(51) Int. Cl.: G01N 33/569, G01N 33/68

(54) **PROCÉDÉ POUR DÉTERMINER LE RISQUE DE SURVENUE D'UNE INFECTION ASSOCIÉE AUX SOINS CHEZ UN PATIENT**
VERFAHREN ZUR BESTIMMUNG DES RISIKOS EINER MIT DER PFLEGE IN ZUSAMMENHANG STEHENDEN INFEKTION BEI EINEM PATIENTEN
METHOD FOR DETERMINING THE RISK OF INCIDENCE OF A CARE-ASSOCIATED INFECTION IN A PATIENT

(30) Priorité: 09.04.2020 FR 2003591
(43) Date de publication de la demande: 15.02.2023
(73) Titulaire: bioMérieux, 69280 Marcy-l'Étoile (FR); Bioaster, 69007 Lyon (FR)
(72) Inventeur: MALLET, François, 69100 VILLEURBANNE (FR); MONNERET, Guillaume, 69008 Lyon (FR); MOUCADEL, Virginie, 38360 Sassenage (FR); PACHOT, Alexandre, 01400 Sulignat (FR); PERONNET, Estelle, 69009 Lyon (FR); TEXTORIS, Julien, 69100 VILLEURBANNE (FR); VENET, Fabienne, 69008 Lyon (FR); RIMMELÉ, Thomas, 69007 Lyon (FR)
(74) Mandataire: Germain Maureau
(86) Numéro de dépôt international: PCT/FR2021/050615
(87) Numéro de publication internationale: WO 2021/205121

(56) Documents cités:
- WO-A1-2018/202864
- JENNIFER A MUSZYNSKI ET AL: "Early adaptive immune suppression in children with septic shock: a prospective observational study", CRITICAL CARE, BIOMED CENTRAL LTD LONDON, GB, vol. 18, no. 4, 8 July 2014 (2014-07-08), pages R145: 1 - 10, XP021192557, ISSN: 1364-8535, DOI: 10.1186/CC13980
- JENNIFER A. MUSZYNSKI ET AL: "Innate Immune Function Predicts the Development of Nosocomial Infection in Critically Injured Children", SHOCK, vol. 42, no. 4, 1 October 2014 (2014-10-01), US, pages 313 - 321, XP055761476, ISSN: 1073-2322, DOI: 10.1097/SHK.0000000000000217
- KRISTIN C GREATHOUSE ET AL: "Abstract 16525: Early Innate and Adaptive Immune Suppression Predicts Complications After Pediatric Cardiac Surgery", CIRCULATION, vol. 138, no. Suppl. 1, 5 November 2018 (2018-11-05), US, XP055761507, ISSN: 1524-4539, DOI: 10.1161/circ.138.suppl_1.16525

## Description

### DOMAINE DE L'INVENTION

La présente description concerne un procédé *in vitro* ou *ex vivo,* basé sur la mesure de l'expression de cytokine(s), à partir d'un échantillon sanguin d'un patient, incubé avec un stimulus, pour déterminer le risque de survenue d'une infection associée aux soins chez ledit patient, dans les sept jours suivant le jour où le prélèvement de l'échantillon sanguin a été effectué sur ledit patient.

### TECHNIQUE ANTERIEURE

Le développement d'infections associées aux soins est une complication importante liée aux soins médicaux, en particulier dans les structures de soins médicalisées comme les hôpitaux (où l'on parlera plus précisément d'infections nosocomiales). Il a été montré que les infections nosocomiales en unités de soins intensifs, qui surviennent chez 20 à 40% des patients, étaient associées à une morbidité et une mortalité accrues, une durée plus longue du besoin en soins de suppléance aux défaillances d'organe(s), une durée de séjour plus longue à l'hôpital, des coûts de santé plus importants, et une utilisation plus importante d'antibiotiques, contribuant à la résistance antimicrobienne. L'occurrence des infections associées aux soins est particulièrement exacerbée depuis quelques années, de par l'augmentation des pathogènes multirésistants. L'Organisation Mondiale de la Santé (OMS) estime à environ 5 millions le nombre d'infections nosocomiales dans les hôpitaux en Europe, conduisant à environ 50 000 morts et un surcoût annuel de 13 à 24 milliards d'euros. De nombreux facteurs influent sur la survenue et le développement des infections associées aux soins, comme l'état général de santé du patient, mais également des facteurs liés à la prise en charge du patient (e.g. l'administration d'antibiotiques et/ou l'utilisation de dispositifs médicaux invasifs), des facteurs liés à l'environnement hospitalier (e.g. le ratio du nombre d'infirmières par rapport au nombre de patients), et l'utilisation variable des techniques aseptiques par le personnel hospitalier. Des recommandations ont été publiées, et la mise en place de programmes de contrôle des infections a été encouragée, en particulier par le *U.S. Department of Health and Human Services,* le Centre européen pour la prévention et le contrôle des maladies, l'OMS et les agences nationales, pour lesquels la prévention et la réduction des infections associées aux soins sont devenues une priorité majeure. Il a été montré que les programmes de contrôle des infections associées aux soins se révèlent particulièrement efficaces pour réduire les infections sévères. Cependant, il a été estimé qu'un maximum de 65 à 70% des cas d'infections du sang et des voies urinaires, liées à la pose de cathéters, et de 55% des cas de pneumonie associées à la ventilation mécanique et d'infections au niveau du site chirurgical, pourraient être évités. Par ailleurs, l'observance et l'application des procédures selon les recommandations peuvent être compliquées dans certains hôpitaux, particulièrement dans les pays à faibles ou moyens revenus. L'identification précoce des patients à risque de développer une infection associée aux soins serait une étape-clé dans la prévention de ces infections et la prise en charge de ces patients. D'après certaines modélisations, un test qui réduirait le temps d'identification des patients à haut risque de contracter des infections associées aux soins, permettrait de réduire la mortalité chez ces patients, avec un bon rapport coût/efficacité. Cependant, il n'existe actuellement pas en clinique de test de diagnostic *in vitro* permettant d'identifier les patients à haut risque de contracter une infection associée aux soins.

Les tests fonctionnels, ou tests fonctionnels immunitaires (IFA, *Immune Functional Assays*), sont des tests mesurant directement, *ex vivo,* la capacité d'une ou plusieurs population(s) cellulaire(s) à répondre à un stimulus avec lequel les cellules sont mises en contact. Ces tests fonctionnels ont par exemple été utilisés en recherche pour étudier l'anergie des monocytes, le plus souvent en mesurant le TNFa au niveau protéique après une stimulation *ex vivo* avec du lipopolysaccharide (LPS), ainsi qu'en clinique, dans le cas de la tuberculose, en mesurant l'interféron y (IFNy) au niveau protéique après une stimulation avec un antigène de *Mycobacteria tuberculosis.* Des tests fonctionnels ont également été utilisés dans le cadre d'une étude visant à définir les limites d'une réponse immunitaire normale (*i*.*e*. en contexte « sain ») en réponse à différents challenges infectieux (Urrutia et al (2016), Cell Reports 16 : 2777-2791).

En outre, il a précédemment été montré, chez des patients atteints de sepsis suite à une chirurgie viscérale majeure, que la sécrétion d'IL2, de TNFa et en partie d'IFNy par des lymphocytes T, stimulés avec des anticorps anti-CD3/CD28, était significativement diminuée chez les patients non-survivants, par rapport aux patients survivants et aux contrôles ; cependant, aucun lien avec le risque de contracter une infection associée aux soins n'y était évoqué (Heidecke et al (2000), Chirurg. 71(2) : 159-65).

D'autre part, certaines études ont montré que le niveau de cytokines sécrétées dans des échantillons de sang provenant de patients pédiatriques (en choc septique, atteints de blessures sévères ou ayant reçu une chirurgie cardiaque) et préalablement incubés avec un stimulus, présentait certaines différences entre les patients qui développaient une infection nosocomiale et ceux qui n'en développaient pas (Muszynski et al (2014), Crit Care 18 : R145 ; Muszynski et al (2014), Shock 42(4) : 313-321 ; Greathouse et al (2018), Circulation 138 : A16525). Les procédés utilisés permettaient d'évaluer soit la fonction immunitaire innée (en utilisant le LPS comme stimulus), soit la fonction immunitaire adaptative (en utilisant la phytohémagglutinine (PHA) comme stimulus). Le LPS se lie au récepteur TLR4, plus particulièrement au niveau de cellules de l'immunité innée, tandis que la PHA est une lectine se liant aux lymphocytes T. Cependant, il est généralement question dans ces études de survenue d'une infection associée aux soins dans les quatorze à trente jours suivant le jour de l'agression (i.e. début du choc septique, blessure ou chirurgie), alors qu'une prédiction plus précoce serait préférable, car la décision en terme d'intervention thérapeutique doit être prise rapidement, de préférence dans la semaine. Il existe donc un besoin de développer des tests permettant de déterminer le risque de survenue d'une infection associée aux soins chez un patient, dans la semaine suivant le jour du prélèvement de l'échantillon de ce patient, c'est-à-dire dans les sept jours suivant ce prélèvement.

Or, il a été découvert que, de façon tout-à-fait surprenante, des tests fonctionnels basés sur la mesure de l'expression de cytokine(s), à partir d'un échantillon sanguin d'un patient, incubé avec certains types de stimuli (tels que des superantigènes, qui lient simultanément des cellules de l'immunité innée et des cellules de l'immunité adaptative), permettaient de déterminer le risque de survenue d'une infection associée aux soins chez ce patient, dans les sept jours suivant le jour où le prélèvement de l'échantillon sanguin a été effectué sur ledit patient. Ces patients à risque de contracter une infection associée aux soins pourraient avantageusement bénéficier d'une prise en charge individualisée ou d'un traitement immunostimulant.

### RESUME DE L'INVENTION

L'invention est décrite dans le jeu de revendications annexé et a notamment pour objet un procédé *in vitro* ou *ex vivo* pour déterminer le risque de survenue d'une infection associée aux soins, de préférence une infection nosocomiale, chez un patient, de préférence un patient au sein d'un établissement de santé, dans les sept jours suivant le jour où le prélèvement de l'échantillon sanguin a été effectué sur ledit patient, comprenant :
a) Une étape d'incubation d'un échantillon sanguin du patient avec un stimulus, ledit stimulus comprenant une molécule choisie dans le groupe constitué par :
   - une molécule e type superantigène ou une molécule analogue à un superantigène, et
   - un ou plusieurs anticorps permettant une activation directe des lymphocytes T choisi(s) parmi les anticorps reconnaissant et activant un récepteur à la surface du lymphocyte T, et
b) Une étape de mesure de l'expression, à partir de l'échantillon sanguin stimulé résultant de l'étape a), d'au moins une cytokine, ladite cytokine étant produite par des cellules de l'immunité innée et/ou par des cellules de l'immunité adaptative.

Bien entendu, le procédé comprend une dernière étape de détermination du risque de survenue de ladite infection.

### DESCRIPTION DETAILLEE

Dans le cadre de la présente description :
- Le terme « patient » désigne un individu (être humain), qui est entré en contact avec un professionnel de santé, tel qu'un médecin (par exemple, un médecin généraliste) ou une structure médicale ou un établissement de santé (par exemple, un hôpital, et plus particulièrement le service des urgences, le service de réanimation, une unité de soins intensifs ou une unité de soins continus, ou une structure médicalisée pour personnes âgées, de type EHPAD). Le patient peut par exemple être une personne âgée, dans le cadre d'un protocole de vaccination (notamment en EHPAD ou encore chez un médecin généraliste) ;
- Une infection est dite « associée aux soins », si elle survient au cours ou au décours d'une prise en charge (diagnostique, thérapeutique, palliative, préventive, éducative ou opératoire) d'un patient par un professionnel de santé, et si elle n'était ni présente, ni en incubation au début de la prise en charge. Les infections associées aux soins (IAS) comprennent les infections contractées au sein d'un établissement de santé (dites infections nosocomiales) mais aussi lors de soins délivrés hors de ce cadre. Lorsque l'état infectieux au début de la prise en charge n'est pas connu précisément, un délai d'au moins 48 heures ou un délai supérieur à la période d'incubation est couramment accepté pour définir une IAS. Pour les infections du site opératoire, on considère habituellement comme associées aux soins les infections survenant dans les trente jours suivant l'intervention ou, s'il y a mise en place d'un implant, d'une prothèse ou d'un matériel prothétique dans l'année qui suit l'intervention ;

- Un « échantillon sanguin » désigne un échantillon de sang total ou un échantillon cellulaire dérivé du sang (i.e. un échantillon obtenu à partir du sang et contenant au moins un type de cellules, tel qu'un échantillon de cellules mononuclées du sang périphérique ou PBMC) ;
- Par « cellule présentatrice d'antigène » (CPA), on entend une cellule du système immunitaire qui présente des parties d'éléments intrus à des lymphocytes T. Il peut s'agir d'une cellule de l'immunité innée (e.g. monocyte, macrophage, cellule dendritique) ou d'un lymphocyte B ;
- Par « analogue d'anticorps », on entend une molécule mimant un comportement de reconnaissance paratope/épitope, tel qu'avec les anticorps. Ces analogues d'anticorps sont bien connus de l'homme du métier. A titre d'exemples, on peut citer les Fab, Fab', F(ab')₂, scFv, les nanobodies, les adnectines/monobodies, les diabodies, les affibodies, les aptamères, les anticalines, les DARPins, les avimères, les affilines, les fynomères, les nanofitines/affitines, les knottines, les pronectines, les domaines de Kunitz.

Une infection associée aux soins survenant dans les sept jours suivant le jour où le prélèvement de l'échantillon sanguin a été effectué sur le patient, peut survenir au cours du 1^{er} jour, du 2^{ème} jour, du 3^{ème} jour, du 4^{ème} jour, du 5^{ème} jour, du 6^{ème} jour ou du 7^{ème} jour suivant le jour où le prélèvement de l'échantillon sanguin a été effectué sur le patient (quel que soit le jour où ce prélèvement a été effectué).

De préférence, dans le procédé tel que décrit précédemment, le patient est un patient au sein d'un établissement de santé, de préférence au sein d'un hôpital, de préférence encore un patient au sein du service des urgences, d'un service de réanimation, en unité de soins intensifs ou en unité de soins continus ; de préférence encore, un patient ayant subi une agression inflammatoire sévère ; de préférence encore, un patient en état septique (et plus particulièrement, un patient en choc septique, et notamment un patient ayant besoin de vasopresseurs et/ou dont le lactate dépasse les 2 mmol/L), un patient atteint de brûlures (et plus particulièrement, de brûlures graves), un patient atteint de traumatismes (et plus particulièrement, de traumatismes graves), ou un patient opéré par chirurgie (et plus particulièrement, une chirurgie lourde). Dans ce cas, le procédé selon l'invention permet de déterminer le risque de survenue d'une infection nosocomiale chez le patient. Par patient en état septique (ou patient atteint de sepsis), on entend un patient présentant au moins une défaillance d'organe menaçant le pronostic vital et causée par une réponse inappropriée de l'hôte à une infection. Par choc septique, on entend un sous-type de sepsis, dans lequel une hypotension persiste, malgré un remplissage vasculaire adéquat. Dans le cas d'un patient en état septique (déjà atteint d'une première infection), le procédé selon l'invention permet de déterminer le risque de survenue d'une infection secondaire.

Les patients peuvent être des patients pédiatriques (âgés de 18 ans ou moins) ou des patients adultes (âgés de plus de 18 ans) ; de préférence, il s'agit de patients adultes.

De préférence, le procédé tel que décrit précédemment, dans tous ses modes de réalisation, est appliqué à un échantillon sanguin contenant des leucocytes, en particulier contenant au moins des lymphocytes T. L'échantillon sanguin peut par exemple être un échantillon de lymphocytes T purifiés. Il peut également s'agir d'un échantillon de cellules mononuclées du sang périphérique (ou PBMC, *Peripheral Blood Mononuclear Cells*), qui est constitué des lymphocytes (B, T et cellules NK), des cellules dendritiques et des monocytes, et qui est généralement obtenu par la méthode Ficoll, bien connue de l'homme du métier. Cependant, on préfèrera utiliser directement un échantillon de sang total (c'est-à-dire contenant l'ensemble des leucocytes, érythrocytes, plaquettes et le plasma), tel que collecté par la voie veineuse (par exemple en utilisant des tubes contenant un anticoagulant), afin de minimiser les manipulations de l'échantillon et de préserver les interactions cellulaires physiologiques entre les différentes populations cellulaires impliquées dans la réponse immunitaire, et de mieux refléter la complexité des réponses immunitaires innées et adaptatives chez le patient. En particulier, alors que les PBMC ne contiennent que les cellules mononuclées, le sang total contient également des granulocytes (ou polynucléaires).

Le prélèvement de l'échantillon sanguin peut avoir été réalisé le cas échéant à l'admission dans un établissement de santé ou au décours de l'évolution du patient, notamment lors de la première semaine (i.e. à J1, J2, J3, J4, J5, J6 ou J7) après l'agression inflammatoire ou après l'admission.

Le procédé selon l'invention repose sur un test fonctionnel, dans lequel un échantillon sanguin du patient est incubé avec un stimulus. En particulier, ce stimulus se lie spécifiquement à une cellule de l'immunité adaptative (plus particulièrement, un lymphocyte T, notamment au niveau des chaînes variables alpha et beta des récepteurs des cellules T ou TCR) et possiblement également à une cellule présentatrice d'antigène (notamment au niveau du complexe majeur d'histocompatibilité de classe Il ou CMH II).

Ainsi, dans le procédé selon l'invention, dans tous ses modes de réalisation, le stimulus comprend une molécule choisie dans le groupe constitué par (i) une molécule de type superantigène ou une molécule analogue à un superantigène, et (ii) un ou plusieurs anticorps permettant une activation directe des lymphocytes T choisi(s) parmi les anticorps reconnaissant et activant un récepteur à la surface du lymphocyte T.

Les superantigènes sont des toxines de nature protéique, capables de stimuler un grand nombre de lymphocytes T, par l'intermédiaire de leur liaison simultanée à la chaîne β du domaine variable (V_{β}) d'un récepteur des cellules T *via* la région hypervariable CDR4, et à une molécule du CMH Il (complexe majeur d'histocompatibilité de classe II), présente en surface d'une cellule présentatrice d'antigène (CPA). L'interaction forcée qui s'établit entre la cellule présentatrice d'antigène portant le CMH et les lymphocytes T dont le récepteur des cellules T porte le segment V_{β}, provoque une activation polyclonale de ces lymphocytes T, indépendamment de leur spécificité pour l'antigène peptidique présenté. Lorsqu'un stimulus comprenant une molécule de type superantigène est utilisé, l'échantillon sanguin utilisé dans le procédé selon l'invention, contient de préférence des lymphocytes T et des cellules présentatrices d'antigène. Parmi les superantigènes plus particulièrement d'intérêt, on peut notamment citer les superantigènes produits par les espèces staphylococciques et les superantigènes produits par les espèces streptococciques. Les superantigènes de staphylocoques et de streptocoques sont connus. A titre d'exemples de superantigènes de staphylocoques, on peut citer SEA, SEB, SEC (incluant notamment les variants SEC1, SEC2, SEC3 et SEC4), SED, SEE, SEF, SEG, SEH, SEI, SEJ, SEK, SEL, SEM, SEN, SEO, SEP, SEQ, SER, SES, SET, SEU, SEV (*Staphylococcal Enterotoxin* A à V) et TSST-1, et à titre d'exemples de superantigènes de streptocoques, on peut citer SPE A, SPE B et SPE C (*Streptococcal Pyogenic Exotoxin* A à C). De préférence, le stimulus comprend au moins une molécule choisie parmi SEA (*Staphylococcal Enterotoxin A*), SEB (*Staphylococcal Enterotoxin B*) et SEC (*Staphylococcal Enterotoxin C).* Parmi les molécules analogues à un superantigène, on peut par exemple citer des anticorps bispécifiques, capables de se lier d'une part à un lymphocyte T, et d'autre part à une cellule présentatrice d'antigène (comme, par exemple, des anticorps capables de se lier d'une part au V_{β} sur les lymphocytes T, et, d'autre part à une molécule du CMHII ou à un récepteur de type TLR, sur les cellules présentatrices d'antigène).

Dans le procédé selon l'invention, on peut également utiliser un stimulus comprenant un ou plusieurs anticorps permettant une activation directe des lymphocytes T, en particulier *via* la reconnaissance et l'activation d'un récepteur à la surface du lymphocyte T. Il peut s'agir d'anticorps étant associés physiquement et/ou chimiquement entre eux, de préférence encore par couplage sur polymères, par couplage sur billes, par couplage sur de la BSA (*Bovine Serum Albumine*) ou par couplage entre eux. De préférence, il peut s'agir d'anticorps anti-CD3 (tel que le Muromonab-CD3, commercialisé sous le nom Orthoclone OKT3), qui se lient aux lymphocytes T, de préférence encore lesdits anticorps anti-CD3 étant associés physiquement et/ou chimiquement avec un ou plusieurs anticorps, pouvant de préférence être sélectionnés dans la liste constituée par : des anticorps anti-HLA-DR (*Human Leukocyte Antigen-DR*), des anticorps anti-CD28, des anticorps anti-CD2 et/ou des anticorps anti-CD137/TNFRSF9, lesquels se lient à des cellules présentatrices d'antigène, comme indiqué précédemment.

Il est particulièrement avantageux d'utiliser, dans le cadre du procédé selon l'invention, des systèmes permettant une standardisation des procédures ; en particulier, on pourra utiliser des systèmes de culture semi-fermés (e.g. des tubes) pré-remplis avec le milieu de culture et le stimulus d'intérêt, qui sont standardisés, e.g. qui contiennent un stimulus bien défini (i.e. sans variabilité inter-lots au niveau de la production du stimulus, quant à sa nature/sa composition) et/ou chargés en « batch », de manière à contrôler la quantité de stimulus dans le tube et avoir une reproductibilité de tube à tube. De préférence, cestubes peuvent également permettre la collecte de l'échantillon de sang (ce qui permet de stimuler les cellules au moment de la collecte), et de préférence encore, ils permettent la collecte d'un volume précis de sang. On peut citer à titre d'exemple de systèmes standardisés les tubes TruCulture^{®}.

Dans le procédé tel que décrit précédemment, dans tous ses modes de réalisation, l'étape d'incubation de l'échantillon sanguin du patient avec le stimulus peut être réalisée à différentes températures (préférentiellement à 37°C) et à différents temps d'incubation (préférentiellement entre 1h et 48h d'incubation ; par exemple avec une incubation d'1h ou moins, de 2h ou moins, de 4h ou moins, de 12h ou moins, de 24h ou moins, ou de 48h ou moins). Des temps d'incubation courts sont particulièrement avantageux pour la mise en oeuvre du test en clinique.

L'incubation de l'échantillon sanguin du patient avec un stimulus, tel que décrit précédemment, induit une réponse cellulaire résultant en la production de cytokine(s), qui permet de déterminer le risque de survenue d'une infection associée aux soins chez un patient, dans les sept jours suivant le jour où le prélèvement de l'échantillon sanguin a été effectué sur ledit patient.

Ainsi, dans le procédé tel que décrit précédemment, dans tous ses modes de réalisation, on mesure l'expression d'au moins une cytokine, ladite cytokine étant produite par des cellules de l'immunité innée et/ou par des cellules de l'immunité adaptative.

Un faible niveau d'expression de cytokine(s) est associé à un risque plus élevé de contracter une infection associée aux soins, dans les sept jours suivant le prélèvement de l'échantillon sanguin. Il peut s'agir de mesurer l'expression d'au moins une, au moins deux, au moins trois, au moins quatre, au moins cinq, au moins six, au moins sept, au moins huit, au moins neuf, au moins dix cytokine(s) choisie(s) dans le groupe constitué par GM-CSF, IFNy, IL2, IL3, IL4, IL5, IL6, IL10, IL17 et TNFa (liste « immunité adaptative »), de préférence choisie(s) dans le groupe constitué par GM-CSF, IFNy, IL2, IL3, IL4, IL5, IL6, IL10 et IL17, de préférence encore choisie(s) parmi IFNy et IL2. Ces cytokines correspondent à des cytokines produites par des cellules de l'immunité adaptative (en particulier, des lymphocytes T). Il peut également s'agir de mesurer l'expression d'au moins une, au moins deux, au moins trois, au moins quatre, au moins cinq, au moins six, au moins sept, au moins huit, au moins neuf, au moins dix, au moins onze, au moins douze, au moins treize, au moins quatorze cytokine(s) choisie(s) dans le groupe constitué par CCL2 (MCP1), CCL3 (MIP1 alpha), CCL4 (MIP1 beta), CXCL8 (IL8), CXCL10 (IP10), IFNy, IL1α, IL1β, IL1RA, IL3, IL6, IL10, IL18 et TNFa (liste « immunité innée »), de préférence encore ladite au moins une cytokine étant IFNy. Ces cytokines correspondent à des cytokines produites par des cellules de l'immunité innée (en particulier, des monocytes et/ou des macrophages).

Il peut encore s'agir de mesurer l'expression d'au moins deux, au moins trois, au moins quatre, au moins cinq, au moins six, au moins sept, au moins huit, au moins neuf, au moins dix, au moins onze, au moins douze, au moins treize, au moins quatorze, au moins quinze, au moins seize, au moins dix-sept, au moins dix-huit, au moins dix-neuf cytokines différentes, respectivement choisies dans la liste « immunité innée » et dans la liste « immunité adaptative », de préférence au moins une cytokine parmi les au moins deux cytokines différentes étant choisie parmi IFNy et IL2, de préférence encore lesdites deux cytokines différentes étant IFNy et IL2.

La mesure de l'expression de cytokine(s) peut en particulier être réalisée au niveau protéique. Les techniques permettant un telle mesure de l'expression protéique sont bien connues de l'homme du métier. A titre d'exemples, on peut citer les dosages par immunoessais, tels qu'ELISA (*Enzyme Linked ImmunoSorbent Assay*), ELFA (*Enzyme Linked Fluorescent Assay*) et RIA (*radio immunoassays*), par ECL (Electrochimiluminescence) et les dosages par spectrométrie de masse.

De préférence, le procédé tel que décrit précédemment, dans tous ses modes de réalisation, peut comprendre également une étape de mesure de l'expression, à partir d'un échantillon sanguin contrôle sans stimulation (c'est-à-dire l'échantillon sanguin incubé dans les mêmes conditions que l'échantillon sanguin stimulé, mais en l'absence de stimulus) de la (des) même(s) cytokine(s) que celle(s) mesurée(s) à partir de l'échantillon sanguin stimulé. De préférence encore, le procédé peut comprendre en outre une étape de calcul des ratios de l'expression de chaque cytokine dans l'échantillon sanguin stimulé, par rapport à l'expression de la même cytokine dans l'échantillon sanguin contrôle (non stimulé), ou une étape de soustraction de l'expression de chaque cytokine dans l'échantillon sanguin contrôle (non stimulé) à l'expression de la même cytokine dans l'échantillon sanguin stimulé.

De préférence, dans le procédé tel que décrit précédemment, dans tous ses modes de réalisation, l'expression de la(des) cytokine(s) dans l'échantillon sanguin stimulé du patient est comparée à une valeur de référence ou à l'expression de la(des) même(s) cytokine(s) dans un échantillon sanguin de référence. L'échantillon sanguin de référence peut être par exemple un échantillon sanguin provenant d'un volontaire (individu sain), un mélange d'échantillons provenant de plusieurs volontaires (individus sains), un échantillon d'un patient ou un mélange d'échantillons provenant de plusieurs patients, le(s) patient(s) pouvant en particulier avoir subi une agression inflammatoire sévère, tel que décrit précédemment ; ces échantillons sanguins de référence étant de préférence stimulés par le même stimulus que l'échantillon sanguin stimulé du patient à tester.

De préférence, dans le procédé tel que décrit précédemment, dans tous ses modes de réalisation, le ratio de l'expression de chaque cytokine dans l'échantillon sanguin stimulé, par rapport à l'expression de la même cytokine dans l'échantillon sanguin contrôle (non stimulé), est comparé à une valeur de référence correspondant au ratio de l'expression de la même cytokine dans un échantillon stimulé issu de l'échantillon sanguin de référence, par rapport à l'expression de la même cytokine dans un échantillon non stimulé issu de l'échantillon sanguin de référence.

De préférence, dans le procédé tel que décrit précédemment, dans tous ses modes de réalisation, la valeur résultant de la soustraction de l'expression de chaque cytokine dans l'échantillon sanguin contrôle (non stimulé) à l'expression de la même cytokine dans l'échantillon sanguin stimulé est comparée à une valeur de référence résultant de la soustraction de l'expression de la même cytokine dans un échantillon non stimulé issu de l'échantillon sanguin de référence à l'expression de la même cytokine dans un échantillon stimulé issu de l'échantillon sanguin de référence.

Le test fonctionnel selon l'invention peut être utilisé seul, ou bien combiné à certains paramètres immunitaires, de manière à améliorer la prédiction de survenue d'une infection associée au soins dans les sept jours suivant le prélèvement de l'échantillon sanguin. Ainsi, le procédé tel que décrit précédemment, dans tous ses modes de réalisation, peut également comprendre une étape de mesure, dans un échantillon sanguin du patient n'ayant pas été incubé avec un stimulus, de la concentration d'IL10, de la concentration d'IL6, du nombre de molécules d'HLA-DR par monocyte, du pourcentage de neutrophiles CD10^{low}/CD16^{low} et/ou du pourcentage de neutrophiles CD10^{high}/CD16^{high}.

L'invention a également pour objet l'utilisation :
- de moyen(s) de détection de l'expression d'au moins une, au moins deux, au moins trois, au moins quatre, au moins cinq, au moins six, au moins sept, au moins huit, au moins neuf, au moins dix, au moins onze, au moins douze, au moins treize, au moins quatorze, au moins quinze, au moins seize, au moins dix-sept, au moins dix-huit, au moins dix-neuf cytokine(s) choisie(s) parmi celles décrites précédemment, lesdits moyens de détection étant de préférence des anticorps, ou
- d'un kit comprenant de tels moyens de détection, ledit kit comprenant de préférence, en outre, un stimulus selon l'invention tel que défini précédemment,
pour déterminer le risque de survenue d'une infection associée aux soins, de préférence une infection nosocomiale, chez un patient, de préférence un patient au sein d'un établissement de santé, dans les sept jours suivant le jour où le prélèvement de l'échantillon sanguin, à partir duquel l'expression de cytokine(s) est mesurée, a été effectué sur ledit patient.

### Figures

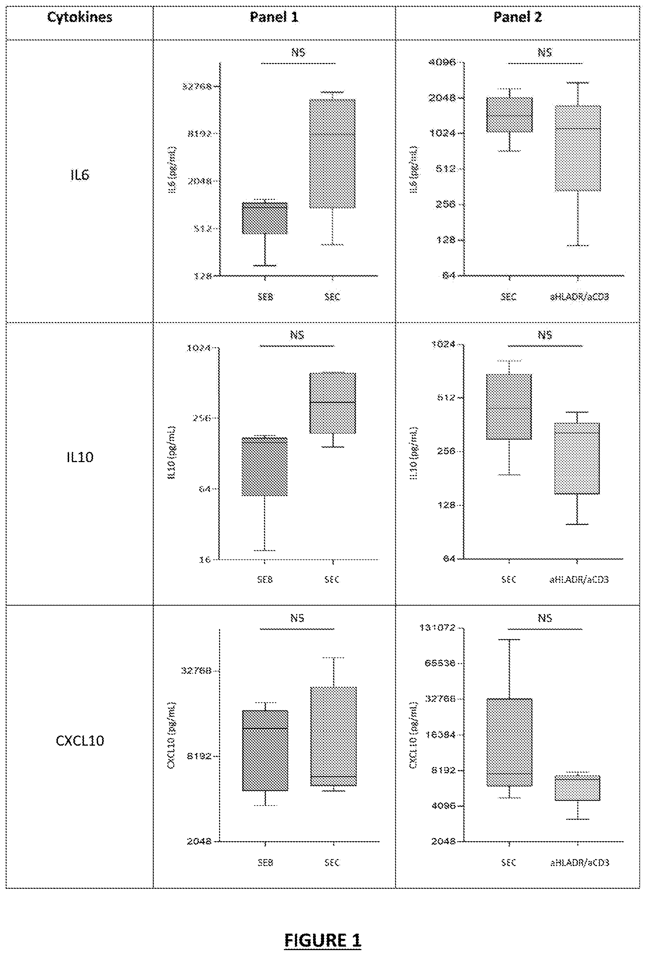

Figure 1 : Effet de différents stimuli (SEB, SEC et un conjugué bifonctionnel 'aHLADR/aCD3' comportant des anticorps monoclonaux anti-HLA-DR et anti-CD3 greffés sur de la BSA) sur la sécrétion des cytokines IL6, IL10 et CXCL10. L'incubation de 100 µL (panel 1) ou 300 µL (panel 2) de sang total avec le stimulus a été effectuée à 37°C pendant 24h (panel 1) ou 16h (panel 2), avant la mesure de la quantité de cytokines sécrétées. « NS » : non statistiquement significatif.

La présente invention est illustrée de manière non limitative par les exemples suivants.

### Exemple 1

### Matériels et Méthodes

Une étude clinique observationnelle prospective, longitudinale et monocentrique a été réalisée à l'Hôpital Edouard Herriot (Lyon, France). Le design de cette étude clinique a été publié dans Rol et al (2017), BMJ Open 7(6) : e015734. L'étude clinique a été approuvée par l'Agence Nationale de Sécurité du Médicament et des produits de santé (ANSM) en novembre 2015 et le Comité de Protection des Personnes Sud-Est Il en décembre 2015. Des amendements au protocole ont été effectués en juillet 2016, puis en janvier 2017. Brièvement, un total de 377 patients, en état septique (n=35) ou en choc septique (n=72), atteints de brûlures graves (n=24), de traumatismes graves (n=137) ou hospitalisés dans un service de réanimation ou une unité de soins intensifs après une chirurgie majeure (n=109), et 175 volontaires sains ont été inclus entre décembre 2015 et mars 2018.
- Patients en état septique/en choc septique : selon le premier protocole clinique, seuls les patients en choc septique étaient inclus, sur la base d'une suspicion d'un foyer infectieux, un début de traitement par catécholamines dans les 48h suivant l'admission en réanimation et d'un traitement au catécholamines (noradrénaline) > 0.25 µg/kg/min pendant au moins 2 heures. Ensuite, les critères d'éligibilité ont été modifiés en août 2016, suite à la publication d'une nouvelle définition du choc septique, Sepsis 3 (Singer et al (2016), JAMA 810-801:(8)315). Les patients en choc septique étaient donc inclus sur la base d'une suspicion d'un foyer infectieux, un début de traitement par catécholamines dans les 48h suivant l'admission en réanimation et d'une thérapie vasopressive nécessaire pour maintenir une pression artérielle ≥ 65 mm Hg et concentration en lactate > 2 mmol/L (18 mg/dL), malgré la correction d'une hypovolémie. En 2017, a été ajoutée la possibilité d'inclure des patients en état septique (selon la définition Sepsis 3), à savoir la suspicion d'un foyer infectieux et l'augmentation du score SOFA ≥ 2 points par rapport au SOFA de base dans les 48h qui suivent l'admission en réanimation. Pour cette population, le jour 1 correspond au jour du diagnostic du sepsis ou du choc septique ;
- Traumatismes graves : dans le premier protocole, seuls les patients avec un traumatisme grave ont été inclus (Injury Severity Score (ISS) ≥ 25). En août 2016, a été ajoutée la possibilité d'inclure également des traumatismes moins graves (16 < ISS < 24). Pour cette population, le jour 1 correspond au jour d'admission en en service de réanimation ou unité de soins intensifs (~jour du traumatisme) ;
- Chirurgie majeure : dans le premier protocole, seules une oesogastrectomie, une résection de la vessie de type Bricker, une duodénopancréatectomie céphalique et chirurgie de l'aorte abdominale par laparotomie ont été considérées. D'autres types de chirurgie à haut risque de complication ont été ajoutés en janvier 2017 : une pancréatectomie (totale ou caudale), des tumeurs neuroendocrines, une hépatectomie (sur le côté droit), une colectomie étendue (laparotomie), une résection abdopérinéale, une néphrectomie (laparotomie, PKD), un pontage ilio-fémoral (Scarpa). Pour cette population, le jour 1 correspond au jour de la chirurgie ;
- Brûlures graves : les patients ont été sélectionnés sur la base d'une surface totale de brûlures supérieure à 30%. Pour cette population, le jour 1 correspond au jour d'admission en service de réanimation ou unité de soins intensifs (~ jour de la brûlure).

Les critères d'exclusion portaient essentiellement sur des facteurs qui auraient pu impacter le statut immunitaire et biaiser les résultats (par exemple : une neutropénie sévère, des traitements corticostéroïdes, une pathologie onco-hématologique...). Chaque événement conduisant à une suspection d'infection associée aux soins, survenant au sein de l'hôpital avant le jour 30 a été revu indépendamment par trois médecins non impliqués dans le recrutement des patients. Vingt-six pourcents des patients ont développé au moins une infection associée aux soins avant le jour 30, ou avant la sortie de l'hôpital.

Des échantillons de sang total hépariné ont été collectés plusieurs fois pour les patients, i.e. 3-4 fois la première semaine (aux jours 1 ou 2 : J1/2, aux jours 3 ou 4 : J3/4 et aux jours 5, 6 ou 7 : J5/7), puis 3 fois à des temps plus tardifs (autour du J14, du J28 et du J60). Ces échantillons ont été distribués dans des tubes TruCulture (Myriad Rbm, Austin, Texas, États-Unis) préchauffés, contenant le milieu seul (« échantillon contrôle ») ou le milieu avec SEB (100 ng/mL) (« échantillon stimulé »). Ces tubes ont ensuite été insérés dans un incubateur à bloc sec et maintenus à 37°C pendant 24 heures. Après incubation, les concentrations d'IFNy ou IL2 ont été mesurées par un test ELISA (Références : SPCKB-CS-000292 et SPCKB-CS-000955, Biotechne, respectivement), en utilisant la plateforme nanofluidique ELLA (ProteinSimple, San José, Californie, Etats-Unis), selon les recommandations du fournisseur.

En ce qui concerne l'analyse des données, l'association entre la sécrétion de cytokines et le risque de survenue d'une infection associée aux soins a été évaluée pour différents intervalles de temps de survenue de l'infection (i.e. délais entre le prélèvement d'échantillon et la première survenue d'une infection). Les différents délais considérés étaient : une infection associée aux soins dans les 4 jours et dans les 7 jours après le prélèvement d'échantillon, quel que soit le moment où l'échantillon a été prélevé. Pour chaque patient ayant développé une infection associée aux soins (i.e. « patients cas »), l'échantillon considéré correspond au plus proche prélèvement (avec un délai minimum de 24h) avant la survenue du premier épisode d'infection associée aux soins. Pour les patients n'ayant pas développé d'infection associée aux soins (i.e. « patients contrôles »), une méthode d'appariement a été utilisée pour sélectionner pour chaque patient cas, un patient contrôle ayant le même jour de prélèvement de l'échantillon, et des scores SOFA et Charlson proches. Finalement, un unique contrôle a été sélectionné pour chaque cas unique. Des régressions logistiques univariées ont été implémentées. L'analyse a été faite tous types de patients confondus. L'association entre la sécrétion de cytokines et l'occurrence d'une infection associée aux soins a été estimée sous la forme *d'Odds Ratios* exprimés en distance inter-quartile (OR IQR) avec l'intervalle de confiance à 95% qui lui est associé.

### Résultats

Il a été observé qu'une concentration moins élevée d'IL2 ou IFNγ après une stimulation par SEB était associée avec un risque plus élevé de survenue d'une infection associée aux soins dans les 4 ou 7 jours suivant le prélèvement de l'échantillon (Tableau 1).

**Tableau 1. Association entre la mesure d'IFNy ou IL2 après une stimulation par SEB et le risque de survenue d'une infection associée aux soins dans les 4 ou 7 jours suivant le prélèvement de l'échantillon. Les Odd Ratios (OR IQR) sont exprimés en distance inter-quartile avec l'intervalle de confiance à 95% associé (IC).**

| **Cytokine mesurée** | **Nombre de jours après le prélèvement de l'échantillon jusqu'à la 1^{ère} survenue d'infection associée aux soins** | **OR IQR (IC)** | **P** |
|---|---|---|---|
| IFNγ | 4 jours | 0,65 (0,44-0,88) | 0,014 |
| | 7 jours | 0,63 (0,43-0,86) | 0,009 |
| IL2 | 4 jours | 0,51 (0,30-0,82) | 0,007 |
| | 7 jours | 0,65 (0,42-0,96) | 0,038 |

### Exemple 2

Dans cet exemple, les inventeurs ont comparé l'effet de trois stimuli (SEB, SEC et un conjugué bifonctionnel 'aHLADR/aCD3' comportant des anticorps monoclonaux anti-HLA-DR et anti-CD3 greffés sur de la BSA) sur la sécrétion de différentes cytokines (IL6, IL10 et CXCL10).

### Matériels et Méthodes

Des échantillons de sang total (100 µL ou 300 µL) provenant de volontaires sains (n=5 pour chaque condition) ont été incubés à 37°C pendant 16h ou 24h en présence du stimulus à une concentration d'environ 1,4 10⁻⁸ M (soit 0,0004 g/L de SEB (TruCulture^{®}, Myriad, ref 782-001124), 0,0004 g/L de SEC (Toxin Technology, Inc., ref CT111-SEC1) ou 0,0094 g/L du conjugué aHLADR/aCD3 (Ultra-LEAF Purified anti-human HLA-DR - NA.41 (Ozyme), ref BLE307666 et Ultra-LEAF Purified anti-human CD3 - NA.41 (Ozyme), ref BLE317347)) dans du RPMI. Après incubation, les concentrations (en pg/mL) des cytokines (IL6, IL10, CXCL10 - ProteinSimple) ont été mesurées en utilisant la plateforme nanofluidique ELLA (ProteinSimple). Les comparaisons entre les différents stimuli ont été effectuées en utilisant un test t non paramétrique (test des rangs signés de Wilcoxon sur échantillons appariés). Une valeur de p ≤ 0,05 était considérée comme statistiquement significative (test bilatéral).

### Résultats

La figure 1 montre les résultats obtenus avec les différents stimuli. Le panel 1 illustre qu'il n'y avait pas de différence statistiquement significative au niveau de la sécrétion d'IL6, IL10 et CXCL10, entre une stimulation par SEB et une stimulation par SEC. De manière similaire, le panel 2 montre qu'il n'y a pas de différence statistiquement significative au niveau de la sécrétion de ces cytokines entre une stimulation par SEC et une stimulation par le conjugué aHLADR/aCD3. Ces trois stimuli induisent donc la sécrétion de quantités similaires de cytokines dans des échantillons sanguins.

## Revendications

1. Procédé *in vitro* ou *ex vivo* pour déterminer le risque de survenue d'une infection associée aux soins, de préférence une infection nosocomiale, chez un patient, de préférence un patient au sein d'un établissement de santé, dans les sept jours suivant le jour où le prélèvement de l'échantillon sanguin a été effectué sur ledit patient, comprenant :
a) Une étape d'incubation d'un échantillon sanguin du patient avec un stimulus, ledit stimulus comprenant une molécule choisie dans le groupe constitué par :
- une molécule de type superantigène ou une molécule analogue à un superantigène, et
- un ou plusieurs anticorps permettant une activation directe des lymphocytes T choisi(s) parmi les anticorps reconnaissant et activant un récepteur à la surface du lymphocyte T;
b) Une étape de mesure de l'expression, à partir de l'échantillon sanguin stimulé résultant de l'étape a), d'au moins une cytokine, ladite cytokine étant produite par des cellules de l'immunité innée et/ou par des cellules de l'immunité adaptative.

2. Procédé selon la revendication 1, **caractérisé en ce que** le patient est un patient au sein d'un établissement de santé, de préférence au sein d'un hôpital, de préférence encore un patient au sein du service des urgences, d'un service de réanimation, en unité de soins intensifs ou en unité de soins continus, de préférence encore, un patient ayant subi une agression inflammatoire sévère, de préférence encore un patient en état septique, un patient atteint de brûlures, un patient atteint de traumatismes, ou un patient opéré par chirurgie.

3. Procédé selon la revendication 1 ou 2, **caractérisé en ce que** le patient est un patient adulte, âgé de plus de 18 ans.

4. Procédé selon l'une des revendications 1 à 3, **caractérisé en ce que** l'échantillon sanguin est un échantillon de sang total.

5. Procédé selon l'une des revendications 1 à 4, **caractérisé en ce que** le stimulus comprend une molécule choisie parmi les superantigènes produits par les espèces staphylococciques et les superantigènes produits par les espèces streptococciques.

6. Procédé selon l'une des revendications 1 à 5, **caractérisé en ce que** le stimulus comprend SEA (*Staphylococcal Enterotoxin A*), SEB (*Staphylococcal Enterotoxin B*) ou SEC (*Staphylococcal Enterotoxin C*)*.*

7. Procédé selon l'une des revendications 1 à 4, **caractérisé en ce que** molécule analogue à un superantigène est un anticorps bispécifique.

8. Procédé selon l'une des revendications 1 à 4, **caractérisé en ce que** les anticorps reconnaissant et activant un récepteur à la surface du lymphocyte T sont choisis parmi des anticorps anti-CD3, de préférence lesdits anticorps anti-CD3 étant associés physiquement et/ou chimiquement avec un ou plusieurs anticorps, pouvant de préférence être sélectionnés dans la liste constituée par : des anticorps anti-HLA-DR, des anticorps anti-CD28, des anticorps anti-CD2 et/ou des anticorps anti-CD137/TNFRSF9.

9. Procédé selon l'une des revendications 1 à 8, **caractérisé en ce que** dans l'étape b), on mesure l'expression d'au moins une cytokine choisie dans le groupe constitué par GM-CSF, IFNy, IL2, IL3, IL4, IL5, IL6, IL10, IL17 et TNFa (liste « immunité adaptative »), de préférence choisie dans le groupe constitué par GM-CSF, IFNy, IL2, IL3, IL4, IL5, IL6, IL10 et IL17.

10. Procédé selon l'une des revendications 1 à 9, **caractérisé en ce que** dans l'étape b), on mesure l'expression d'au moins une cytokine choisie parmi IFNy et IL2.

11. Procédé selon l'une des revendications 1 à 8, **caractérisé en ce que** dans l'étape b), on mesure l'expression d'au moins une cytokine choisie dans le groupe constitué par CCL2 (MCP1), CCL3 (MIP1 alpha), CCL4 (MIP1 beta), CXCL8 (IL8), CXCL10 (IP10), IFNy, IL1α, IL1β, IL1RA, IL3, IL6, IL10, IL18 et TNFa (liste « immunité innée »), de préférence encore ladite au moins une cytokine étant IFNy.

12. Procédé selon la revendication 9 ou 10 et la revendication 11, **caractérisé en ce que** dans l'étape b), on mesure l'expression d'au moins deux cytokines différentes, respectivement choisies dans la liste « immunité innée » et dans la liste « immunité adaptative ».

13. Procédé selon la revendication 12, **caractérisé en ce que** l'une des dites au moins deux cytokines différentes est choisie parmi IFNy et IL2, de préférence **en ce que** lesdites au moins deux cytokines sont IFNy et IL2.

14. Procédé selon l'une des revendications 1 à 13, **caractérisé en ce que** l'expression de cytokine(s) est mesurée au niveau protéique.

15. Procédé selon l'une des revendications 1 à 14, **caractérisé en ce que** l'expression de cytokine(s) est mesurée par ELISA (*Enzyme Linked ImmunoSorbent Assay*)*,* ELFA (*Enzyme Linked Fluorescent Assay*)*,* RIA (*radio immunoassays*), ECL (Electrochimiluminescence) ou spectrométrie de masse.

16. Procédé selon l'une des revendications 1 à 15, **caractérisé en ce qu'**il comprend une étape de mesure de l'expression, à partir d'un échantillon sanguin contrôle sans stimulation, de la (des) même(s) cytokine(s) que celle(s) mesurée(s) à partir de l'échantillon sanguin stimulé.

17. Procédé selon la revendication 16, **caractérisé en ce qu'**il comprend une étape de calcul des ratios de l'expression de chaque cytokine dans l'échantillon sanguin stimulé, par rapport à l'expression de la même cytokine dans l'échantillon sanguin contrôle.

18. Procédé selon l'une des revendications 1 à 17, **caractérisé en ce qu'**il comprend en outre une étape de mesure, dans un échantillon sanguin du patient n'ayant pas été incubé avec un stimulus, de la concentration d'IL10, de la concentration d'IL6, du nombre de molécules d'HLA-DR par monocyte, du pourcentage de neutrophiles CD10^{low}/CD16^{low} et/ou du pourcentage de neutrophiles CD10^{high}/CD16^{high}.

19. Utilisation :
(i)
- de moyen(s) de détection de l'expression d'au moins une cytokine telle que définie dans l'une des revendications 1 et 9 à 11, ou d'au moins deux cytokines telles que définies dans la revendication 12 ou 13, lesdits moyens de détection étant de préférence des anticorps, ou
- d'un kit comprenant de tels moyens de détection, et (ii) d'une molécule choisie dans le groupe constitué par :
- une molécule de type superantigène ou une molécule analogue à un superantigène, et
- un ou plusieurs anticorps permettant une activation directe des lymphocytes T choisi(s) parmi les anticorps reconnaissant et activant un récepteur à la surface du lymphocyte T,
ladite molécule étant utilisée comme stimulus, et
ladite molécule étant définie dans l'une des revendications 1 et 5 à 8,
pour déterminer le risque de survenue d'une infection associée aux soins chez un patient, dans les sept jours suivant le jour où le prélèvement de l'échantillon sanguin, à partir duquel l'expression de cytokine(s) est mesurée, a été effectué sur ledit patient.

## Patentansprüche

1. *In-vitro-* oder *Ex*-*vivo*-Verfahren zum Bestimmen des Risikos des Auftretens einer mit der Gesundheitsversorgung assoziierten Infektion, vorzugsweise einer nosokomialen Infektion, bei einem Patienten, vorzugsweise einem Patienten innerhalb einer Gesundheitseinrichtung, innerhalb von sieben Tagen nach dem Tag, an dem die Entnahme der Blutprobe von dem Patienten durchgeführt wurde, umfassend:
a) einen Schritt des Inkubierens einer Blutprobe des Patienten mit einem Stimulus, wobei der Stimulus ein Molekül umfasst, das aus der Gruppe ausgewählt ist, die aus Folgendem besteht:
- einem Molekül des Typs Superantigen oder einem Molekül, das einem Superantigen ähnelt, und
- einem oder mehreren Antikörpern, die eine direkte Aktivierung von T-Lymphozyten ermöglichen, ausgewählt aus Antikörpern, die einen Rezeptor auf der Oberfläche der T-Lymphozyte erkennen und aktivieren;
b) einen Schritt des Messens der Expression von mindestens einem Zytokin stimulierten Blutprobe, die aus Schritt a) resultiert, wobei das Zytokin von Zellen der angeborenen Immunität und/oder von Zellen der adaptiven Immunität produziert wird.

2. Verfahren nach Anspruch 1, **dadurch gekennzeichnet, dass** der Patient ein Patient in einer Gesundheitseinrichtung ist, vorzugsweise in einem Krankenhaus, noch bevorzugter ein Patient in der Notaufnahme, auf der Intensivstation, auf der Intensivpflegestation oder auf der Dauerpflegestation, noch bevorzugter ein Patient, der eine schwere entzündliche Aggression erlitten hat, noch bevorzugter ein Patient in septischem Zustand, ein Patient mit Verbrennungen, ein Patient mit Traumata oder ein Patient, der chirurgisch operiert worden ist.

3. Verfahren nach Anspruch 1 oder 2, **dadurch gekennzeichnet, dass** der Patient ein erwachsener Patient im Alter von über 18 Jahren ist.

4. Verfahren nach einem der Ansprüche 1 bis 3, **dadurch gekennzeichnet, dass** die Blutprobe eine Vollblutprobe ist.

5. Verfahren nach einem der Ansprüche 1 bis 4, **dadurch gekennzeichnet, dass** der Stimulus ein Molekül umfasst, das aus Superantigenen, die von Staphylokokkenarten produziert werden, und Superantigenen, die von Streptokokkenarten produziert werden, ausgewählt ist.

6. Verfahren nach einem der Ansprüche 1 bis 5, **dadurch gekennzeichnet, dass** der Stimulus SEA (*Staphylococcal Enterotoxin A*), SEB (*Staphylococcal Enterotoxin B*) oder SEC *(Staphylococcal Enterotoxin C)* umfasst.

7. Verfahren nach einem der Ansprüche 1 bis 4, **dadurch gekennzeichnet, dass** das Molekül, das einem Superantigen ähnelt, ein bispezifischer Antikörper ist.

8. Verfahren nach einem der Ansprüche 1 bis 4, **dadurch gekennzeichnet, dass** die Antikörper, die einen Rezeptor auf der Oberfläche des T-Lymphozyten erkennen und aktivieren, aus Anti-CD3-Antikörpern ausgewählt sind, wobei vorzugsweise die Anti-CD3-Antikörper, die physikalisch und/oder chemisch mit einem oder mehreren Antikörper assoziiert sind, vorzugsweise aus der Liste ausgewählt werden können, die aus Folgendem besteht: Anti-HLA-DR-Antikörpern, Anti-CD28-Antikörpern, Anti-CD2-Antikörpern und/oder Anti-CD137/TNFRSF9-Antikörpern.

9. Verfahren nach einem der Ansprüche 1 bis 8, **dadurch gekennzeichnet, dass** in Schritt b) die Expression von mindestens einem Zytokin, das aus der Gruppe ausgewählt wird, die aus GM-CSF, IFN*γ*, IL2, IL3, IL4, IL5, IL6, IL10, IL17 und TNFα (Liste der "adaptiven Immunität") besteht, vorzugsweise aus der Gruppe ausgewählt wird, die aus GM-CSF, IFN*γ*, IL2, IL3, IL4, IL5, IL6, IL10 und IL17 besteht, gemessen wird.

10. Verfahren nach einem der Ansprüche 1 bis 9, **dadurch gekennzeichnet, dass** in Schritt b) die Expression von mindestens einem Zytokin, das aus IFNγ und IL2 ausgewählt wird, gemessen wird.

11. Verfahren nach einem der Ansprüche 1 bis 8, **dadurch gekennzeichnet, dass** in Schritt b) die Expression von mindestens einem Zytokin, das aus der Gruppe ausgewählt wird, die aus CCL2 (MCP1), CCL3 (MIP1 Alpha), CCL4 (MIP1 Beta), CXCL8 (IL8), CXCL10 (IP10), IFN*γ*, IL1*α*, IL1*β*, IL1RA, IL3, IL6, IL10, IL18 und TNFα (Liste der "angeborenen Immunität") besteht, gemessen wird, wobei noch bevorzugter das mindestens eine Zytokin IFNγ ist.

12. Verfahren nach Anspruch 9 oder 10 und Anspruch 11, **dadurch gekennzeichnet, dass** in Schritt b) die Expression von mindestens zwei verschiedenen Zytokinen gemessen wird, die jeweils aus der Liste der "angeborenen Immunität" und aus der Liste der "adaptiven Immunität" ausgewählt werden.

13. Verfahren nach Anspruch 12, **dadurch gekennzeichnet, dass** eines der mindestens zwei verschiedenen Zytokine aus IFN*γ* und IL2 ausgewählt ist, vorzugsweise dadurch, dass die mindestens zwei Zytokine IFN*γ* und IL2 sind.

14. Verfahren nach einem der Ansprüche 1 bis 13, **dadurch gekennzeichnet, dass** die Expression von Zytokin(en) auf Proteinebene gemessen wird.

15. Verfahren nach einem der Ansprüche 1 bis 14, **dadurch gekennzeichnet, dass** die Expression von Zytokin(en) durch ELISA (*Enzyme Linked ImmunoSorbent Assay*), ELFA (*Enzyme Linked Fluorescent Assay*), RIA (*Radioimmunoassays*), ECL (*Elektrochemilumineszenz*) oder Massenspektrometrie gemessen wird.

16. Verfahren nach einem der Ansprüche 1 bis 15, **dadurch gekennzeichnet, dass** es einen Schritt des Messens der Expression desselben Zytokins bzw. derselben Zytokine, die auch in der stimulierten Blutprobe gemessen wurden, aus einer Kontrollblutprobe ohne Stimulation umfasst.

17. Verfahren nach Anspruch 16, **dadurch gekennzeichnet, dass** es einen Schritt des Berechnens der Verhältnisse der Expression jedes Zytokins in der stimulierten Blutprobe im Vergleich zur Expression desselben Zytokins in der Kontrollblutprobe umfasst.

18. Verfahren nach einem der Ansprüche 1 bis 17, **dadurch gekennzeichnet, dass** es ferner einen Schritt des Messens der Konzentration von IL10, der Konzentration von IL6, der Anzahl der HLA-DR-Moleküle pro Monozyt, des Prozentsatzes an CD10^{low}/CD16^{low}-Neutrophilen und/oder des Prozentsatzes an CD10^{high}/CD16^{high}-Neutrophilen in einer Blutprobe des Patienten, die nicht mit einem Stimulus inkubiert worden ist, umfasst.

19. Verwendung:
- (i) von einem oder mehreren Mitteln zum Nachweisen der Expression von mindestens einem Zytokin, das wie in einem der Ansprüche 1 und 9 bis 11 definiert ist, oder von mindestens zwei Zytokinen, die wie in dem Anspruch 12 oder 13 definiert sind, wobei die Nachweismittel vorzugsweise Antikörper sind, oder
- von einem Kit, das derartige Nachweismittel umfasst,
und (ii) von einem Molekül, das aus der Gruppe ausgewählt ist, die aus Folgendem besteht:
- einem Molekül des Typs Superantigen oder einem Molekül, das einem Superantigen ähnelt, und
- einem oder mehreren Antikörpern, die eine direkte Aktivierung von T-Lymphozyten ermöglichen, ausgewählt aus Antikörpern, die einen Rezeptor auf der Oberfläche der T-Lymphozyte erkennen und aktivieren,
wobei das Molekül als Stimulus verwendet wird, und
wobei das Molekül in einem der Ansprüche 1 und 5 bis 8 definiert ist,
zum Bestimmen des Risikos des Auftretens einer mit der Gesundheitsversorgung assoziierten Infektion bei einem Patienten innerhalb von sieben Tagen nach dem Tag, an dem die Entnahme der Blutprobe, aus der die Expression von Zytokin(en) gemessen wird, von dem Patienten durchgeführt wurde.

## Claims

1. An *in vitro* or *ex vivo* method for determining the risk of developing a healthcare-associated infection, preferably a nosocomial infection, in a patient, preferably a patient in a healthcare facility, within seven days following the day on which the blood sample was collected from said patient, comprising:
a) A step of incubating a patient's blood sample with a stimulus, said stimulus comprising a molecule selected from the group consisting of:
- a superantigen-type molecule or a superantigen-like molecule, and
- one or several antibodies enabling the direct activation of T lymphocytes selected from the antibodies that recognize and activate a receptor on the surface of the T lymphocyte;
b) A step of measuring the expression, from the stimulated blood sample resulting from step a), of at least one cytokine, said cytokine being produced by innate immunity cells and/or adaptive immunity cells.

2. The method according to claim 1, **characterized in that** the patient is a patient in a healthcare facility, preferably in a hospital, more preferably a patient in an emergency department, in a resuscitation department, in an intensive care unit or in a continuing care unit, more preferably a patient having suffered a severe inflammatory attack, more preferably a patient in a septic state, a patient with burns, a patient with traumas, or a patient having undergone surgery.

3. The method according to claim 1 or 2, **characterized in that** the patient is an adult patient, aged over 18 years.

4. The method according to any of claims 1 to 3, **characterized in that** the blood sample is a whole blood sample.

5. The method according to any of claims 1 to 4, **characterized in that** the stimulus comprises a molecule selected from the superantigens produced by the staphylococcal species and the superantigens produced by the streptococcal species.

6. The method according to any of claims 1 to 5, **characterized in that** the stimulus comprises SEA (*Staphylococcal Enterotoxin A*), SEB (*Staphylococcal Enterotoxin B*) or SEC *(Staphylococcal Enterotoxin C*)*.*

7. The method according to any of claims 1 to 4, **characterized in that** the superantigen-like molecule is a bispecific antibody.

8. The method according to any of claims 1 to 4, **characterized in that** the antibodies that recognize and activate a receptor at the surface of the T lymphocyte are selected from anti-CD3 antibodies, said anti-CD3 antibodies preferably being physically and/or chemically associated with one or several antibodies which can preferably be selected from the list consisting of: anti-HLA-DR antibodies, anti-CD28 antibodies, anti-CD2 antibodies and/or anti-CD137/TNFRSF9 antibodies.

9. The method according to any of claims 1 to 8, **characterized in that** in step b), the expression of a least one cytokine selected from the group consisting of GM-CSF, IFNy, IL2, IL3, IL4, IL5, IL6, IL10, IL17 and TNFα ("adaptive immunity" list), preferably selected from the group consisting of GM-CSF, IFNy, IL2, IL3, IL4, IL5, IL6, IL10 and IL17 is measured.

10. The method according to any of claims 1 to 9, **characterized in that** in step b), the expression of at least one cytokine selected from IFNy and IL2 is measured.

11. The method according to any of claims 1 to 8, **characterized in that** in step b), the expression of at least one cytokine selected from the group consisting of CCL2 (MCP1), CCL3 (MIP1 alpha), CCL4 (MIP1 beta), CXCL8 (IL8), CXCL10 (IP10), IFNy, IL1α, IL1β, IL1RA, IL3, IL6, IL10, IL18 and TNFα ("innate immunity" list), more preferably said at least one cytokine being IFNy, is measured.

12. The method according to claim 9 or 10 and claim 11, **characterized in that** in step b), the expression of at least two different cytokines, selected respectively from the "innate immunity" list and from the "adaptive immunity" list is measured.

13. The method according to claim 12, **characterized in that** one of said at least two different cytokines is selected from IFNy and IL2, preferably **in that** said at least two cytokines are IFNy and IL2.

14. The method according to any of claims 1 to 13, **characterized in that** the expression of cytokine(s) is measured at the protein level.

15. The method according to any of claims 1 to 14, **characterized in that** the expression of cytokine(s) is measured by ELISA (*Enzyme Llinked Immunosorbent Assay*), ELFA (*EnzymeLinked Fluorescent Assay*), RIA (*radio immunoassays*), ECL (Electrochemiluminescence) or mass spectrometry.

16. The method according to any of claims 1 to 15, **characterized in that** it comprises a step of measuring the expression, from an unstimulated control blood sample, of the same cytokine(s) as that (those) measured from the stimulated blood sample.

17. The method according to claim 16, **characterized in that** it comprises a step of calculating ratios of the expression of each cytokine in the stimulated blood sample, relative to the expression of the same cytokine in the control blood sample.

18. The method according to any of claims 1 to 17, **characterized in that** it further comprises a step of measuring, from a patient's blood sample which has not been subjected to incubation with a stimulus, the concentration of IL10, the concentration of IL6, the number of HLA-DR molecules per monocyte, the percentage of CD10^{low}/CD16^{low} neutrophils and/or the percentage of CD10^{high}/CD16^{high} neutrophils.

19. A use:
(i)
- of means for detecting the expression of at least one cytokine as defined in any of claims 1 and 9 to 11, or of at least two cytokines as defined in claim 12 or 13, said detection means preferably being antibodies, or
- of a kit comprising such detection means,
and (ii) of a molecule selected from the group consisting of:
- a superantigen-type molecule or a superantigen-like molecule, and
- one or several antibodies enabling the direct activation of the T lymphocytes selected from the antibodies that recognize and activate a receptor at the surface of the T lymphocyte,
said molecule being used as a stimulus, and
said molecule being defined in any of claims 1 and 5 to 8,
to determine the risk of developing a healthcare-associated infection in a patient, within seven days following the day on which the blood sample, from which the expression of cytokine(s) is measured, was collected from said patient.
